# EUROPEAN PATENT SPECIFICATION

(11) **EP 3 013 417 B1**
(45) Date of publication and mention of the grant of the patent: **13.11.2019**
(21) Application number: 14742359.4
(22) Date of filing: 25.06.2014
(51) Int. Cl.: A61N 5/06, A61M 21/00

(54) **ILLUMINATION DEVICE FOR ENHANCING NON-IMAGE FORMING RESPONSES**
BELEUCHTUNGSVORRICHTUNG ZUR VERBESSERUNG VON NICHTBILDERZEUGENDEN REAKTIONEN
DISPOSITIF D'ÉCLAIRAGE POUR ACCENTUER DES RÉPONSES DE NON-FORMATION D'IMAGE

(30) Priority: 26.06.2013 US 201361839506 P
(43) Date of publication of application: 04.05.2016
(73) Proprietor: Signify Holding B.V., 5656 AE Eindhoven (NL)
(72) Inventor: SCHLANGEN, Lucas Josef Maria, NL-5656 AE Eindhoven (NL); LUCAS, Robert James, NL-5656 AE Eindhoven (NL); DOBB, Rachel, NL-5656 AE Eindhoven (NL); BROWN, Timothy Matthew, NL-5656 AE Eindhoven (NL)
(74) Representative: Verweij, Petronella Daniëlle
(86) International application number: PCT/IB2014/062572
(87) International publication number: WO 2014/207661

(56) References cited:
- EP-A1- 2 242 335
- WO-A1-2010/035200
- WO-A1-2011/141842
- WO-A2-2012/146256
- US-A1- 2005 015 122

## Description

### BACKGROUND OF THE INVENTION

### 1. Field of the Invention

The present invention pertains to non-image forming responses to visual stimulation, and, in particular, to an illumination device for enhancing non-image forming responses.

### 2. Description of the Related Art

In the last decade the knowledge of human photo-biology has increased tremendously in the sense that it is clear that light radiation administered to a human subject through the eye, in addition to vision, is of major importance in controlling a variety of biological rhythms. Consequently, light radiation has an influence not only on many body functions, but also on mental performance and mood.

Findings show a sensitivity of melatonin suppression for light radiation administered through the eye. Melatonin is a hormone showing a daily cycle and is considered a marker of the phase of the biological rhythm. During daytime, the melatonin level is relatively low. The melatonin level increases in the evening, and reaches a maximum at night before it decreases gradually again to the minimum level during daytime, i.e. in the period a person normally is awake. Melatonin is generally known as a sleeping hormone that influences the alertness of the human subject. Hence, when the melatonin cycle is controlled, the risk on making mistakes because of lack of alertness is decreased. Suppressing melatonin in the natural daily cycle is possible in the usually "dark" hours of the biological rhythm. Normally in this period only artificial illumination is available.

In a 24-hour society many people have to work and drive at night and be alert to perform well and safe, and to sleep well at abnormal hours. Under these conditions many people run an enhanced risk of making mistakes, for example causing car accidents, and/or are likely to suffer from a distorted sleeping behavior.

Sleep inertia and alertness dips are undesired from a performance or safety perspective. In general, sleep inertia persists for 30 minutes after waking up. For pilots and military, this persistence of sleep inertia may delay or even endanger operations.

WO 02/20079 discloses a method of controlling alertness of a human subject and a light source for use in this method. The method comprises exposure of a human subject during an exposure period to suitable light radiation. Experiments have shown that there is a particularly high sensitivity to light in the region of 420-460 nm, i.e., in the blue part of the photo spectrum.

WO 2011 141 842 discloses a device for influencing sleep patterns of a person, wherein light pulses of different wavelength are applied.

However, there is still a need for improved devices and methods to control and enhance non-image forming responses to light.

### SUMMARY OF THE INVENTION

In one embodiment, an illumination device includes a light unit structured to provide an illumination output; and a controller structured to control said light unit to provide the illumination output at a first intensity and to wait a first period of time, increase intensity of the illumination output to at least a factor times the first intensity, wait a second period of time, and decrease intensity of the illumination output, wherein the factor is at least 1.25.

In another embodiment, a method of providing an illumination scheme includes controlling a light unit to provide an illumination output at a first intensity; waiting a first period of time; increasing intensity of the illumination output to at least a factor times the first intensity; waiting a second period of time; and decreasing intensity of the illumination output, wherein the factor is at least 1.25.

In another embodiment, a non-transitory computer readable medium stores one or more programs, including instructions, which when executed by a computer, causes the computer to perform a method of controlling a light unit to provide an illumination scheme. The method includes controlling the light unit to provide an illumination output at a first intensity; waiting a first period of time; increasing intensity of the illumination output to at least a factor times the first intensity; waiting a second period of time; and decreasing intensity of the illumination output, wherein the factor is at least 1.25.

The invention is defined in the claims, other embodiments are well as methods disclosed not constituting a part of the invention.

### BRIEF DESCRIPTION OF THE DRAWINGS

FIG. 1A-4 are time diagrams of illumination schemes in accordance with some exemplary embodiments of the disclosed concept;
FIG. 5 is a schematic diagram of an illumination device in accordance with an exemplary embodiment of the disclosed concept; and
FIGS. 6-9 are flowcharts of methods of providing an illumination scheme in accordance with some exemplary embodiments of the disclosed concept.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

In the human visual system, light is sensed by photoreceptors in the eye. The photoreceptors include rods and cones as well as intrinsically photosensitive retinal ganglion cells (ipRGCs). The ipRGCs include melanopsin and are intrinsically sensitive to light. That is, ipRGCs will respond to light even in the absence of rods and cones. The responses of the photoreceptors are transmitted through the optic nerve to the brain for processing.

Responses to visual stimuli are generally divided into two types, image forming responses and non-image forming (NIF) responses. Image forming responses generally refer to converting the visual stimuli into a visual perception. NIF responses, also referred to as biological responses, are non-visual responses to light. NIF responses include, for example, entrainment of circadian rhythms, pupillary light reflex, and light-induced alertness.

Light is the most important external cue to synchronize the internal biological rhythm and body clock. The central pacemaker of the biological clock (or circadian clock) resides in a small area of the brain denoted as the Supra Chiasmatic Nuclei (SCN). The biological clock helps to time sleep patterns, alertness, mood, physical strength, blood pressure and much more across the day.

Although the ipRGCs play a significant role in generating NIF responses, the rods and cones also contribute to the NIF responses. The contributions of the ipRGCs, rods and cones to the NIF responses can be influenced by manipulating the dynamics of the light exposure, and thus, it is possible to create a light exposure that causes an optimal NIF response.

For example, when the eye is initially exposed to light, the rods primarily provide the "lights on" response. The rods continue to be of relevance during the entire light pulse. Cones can also play a role in signaling irradiance for NIF responses for higher intensities of light. The ipRGCs have a sluggish response to the initial light exposure, but contribute significantly to the NIF response.

Under natural light conditions, such as a sunrise, light is "turned on" and remains on. Under these conditions, the role of cones in signaling irradiance for NIF responses is quickly taken over by the ipRGCs. The ipRGCs then play the primary role in signaling irradiance for NIF responses. However, by artificially modulating light, the role that cones play in signaling irradiance for NIF responses can be increased, thus producing enhanced NIF responses.

It has been shown that the SCN shows enhanced activity when brief flashes of light are included in a steady background illumination. From basic physiology, one might expect that ipRGCs are able to detect gradual changes in irradiance whereas cones are more responsive to abrupt changes in irradiance. As both of these types of photoreceptors having distinct spectral sensitivities, optimal responses from both of them can be elicited through artificially modulating light.

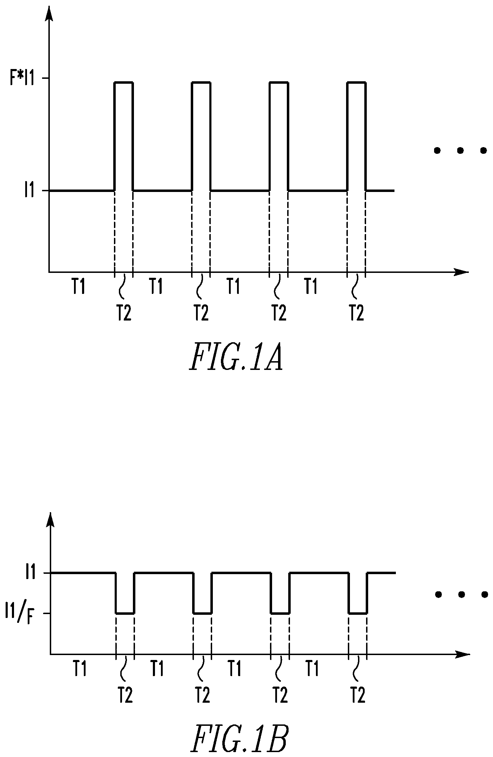

FIG. 1A is a graph of an illumination scheme in accordance with an exemplary embodiment of the disclosed concept. In FIG. 1A, the vertical axis represents intensity of illumination and the horizontal axis represents time. Referring to FIG. 1A, light is provided at a first intensity I1 for a first period of time T1. The light provided during the first period of time T1 is a background illumination and may be, without limitation, white light.

A pulse of light is then provided where the intensity of the light is increased substantially instantaneously to an intensity that is a factor F times the first intensity I1. In other words, the pulse of light has an intensity of F^{∗}I1. The pulse of light is provided during a second period of time T2. At the end of the second period of time T2, the intensity of the light is decreased substantially instantaneously to the first intensity I1. It is also contemplated that at the end of the second period of time T2, the intensity of the light may be decreased to an intensity different than the first intensity I1 such as, without limitation, an intensity that is less than I1.

In one exemplary embodiment of the disclosed concept, factor F is at least 1.25. In another exemplary embodiment of the disclosed concept, factor F is at least 2. In another exemplary embodiment of the disclosed concept, factor F is at least 8. In yet another exemplary embodiment of the disclosed concept, factor F is at least 14. The pulse of light lasts for a second period of time T2 before the intensity returns to the first intensity I1. The sequence of the first and second periods of time are repeated for the duration of the scheme.

In one exemplary embodiment, the spectral composition of the pulse of light includes at least some spectral components having wavelengths within a range of about 400 nm to about 600 nm. This range of wavelengths includes the absorption spectra of the three kinds of cone photoreceptors in humans: the S cones which maximally sensitive to a wavelength of 420 nm; the M cones which are maximally sensitive to a wavelength of 535 nm; and the L cones which are maximally sensitive to a wavelength of 565 nm. The background illumination and the pulse of light have the same spectral composition.

In one exemplary embodiment of the disclosed concept, the pulse of light increases the background illumination by the factor F for at least the portion of the spectrum used for the pulse of light. For example, if the pulse of light is blue and the background illumination has a blue content of 0.1^{∗}I1, then the pulse of light should have an intensity of at least F^{∗}0.1^{∗}I1.

In one exemplary embodiment of the disclosed concept, the pulse of light has an intensity of at least 30 lux, but the disclosed concept is not limited thereto. In some exemplary embodiments of the disclosed concept, the second period of time T2 is, without limitation, within a range of about 0.1 seconds to about 60 seconds. In some exemplary embodiments of the disclosed concept, the second period of time T2 is, without limitation, about 1 second. In some exemplary embodiments of the disclosed concept, the first period of time T1 is, without limitation, at least 3 times greater than second period of time T2. In some exemplary embodiments of the disclosed concept, the second period of time T2 is, without limitation, about 5 seconds.

Over a long period of constant intensity light, the contribution of cones to NIF responses is diminished and taken over by the contribution of ipRGCs. However, the abrupt changes in the intensity of light between the first period of time T1 and the second period of time T2 elicit an enhanced contribution from the cones.

Table 1 shows experimental results obtained when the illumination scheme of FIG. 1A was applied to a mouse. In the experiment, the firing rate of the mouse's SCN was measured to compare the NIF response to the illumination scheme to the NIF response of a steady background illumination. The length of the first period of time T1 was 5 seconds and the length of the second period of time T2 was 1 second. The sequence of the first and second periods of time was repeated ten times. For the experiment, the intensity during the second period of time T2 was varied by different multiples of the first intensity I1.

**Table 1**

| T1 Intensity | T2 Intensity | Change in Firing Rate Relative to Background Firing |
|---|---|---|
| I1 | 2^{∗}I1 | + 30% |
| I1 | 4^{∗}I1 | + 30% |
| I1 | 8^{∗}I1 | + 75% |
| I1 | 14^{∗}I1 | + 105% |
| I1 | 19^{∗}I1 | + 115 % |
| I1 | 24^{∗}I1 | + 120% |

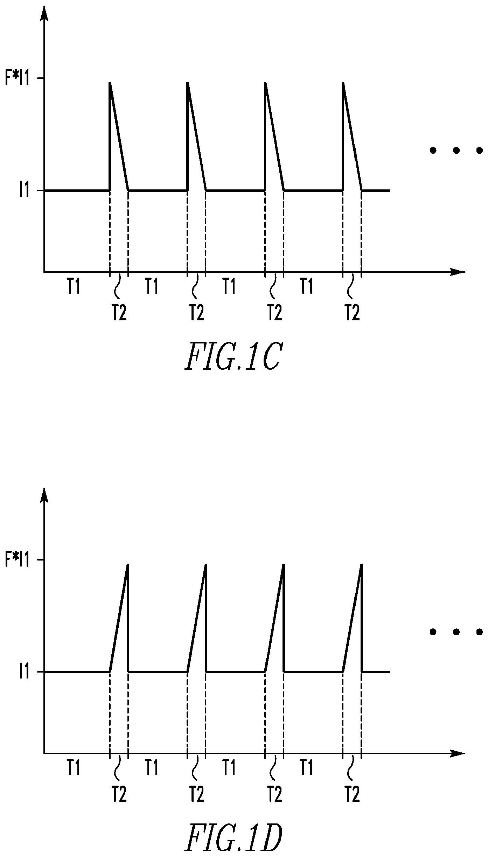

In Table 1, the change in firing rate shows the increase in NIF response compared to the NIF response for steady background illumination. For instance an increase of 120% means the NIF response is 2.2 times the NIF response for steady background illumination. As shown in Table 1, the illumination scheme of FIG. 1 enhances the NIF response considerably. When the second intensity is 14 times the first intensity I1, the NIF response increases 105% over the NIF response from steady background illumination. Further experiments have shown that the enhanced response from the illumination scheme is primarily due to an increased contribution of cones.

FIG. 1B is a graph of an illumination scheme in accordance with an exemplary embodiment of the disclosed concept. In FIG. 1B, the vertical axis represents intensity of illumination and the horizontal axis represents time. Referring to FIG. 1B, light is provided at a first intensity I1 for the first period of time T1. The light provided during the first period of time T1 is a background illumination and may be, without limitation, white light.

At the end of the first period of time T1, the intensity of the light is decreased substantially instantaneously. The intensity of the light is decreased to an intensity that is equal to the first intensity I1 divided by a factor F. The intensity of light remains at the decreased intensity of light for the second period of time T2. At the end of the second period of time T2, the intensity of light is substantially instantaneously increased back to the first intensity I1. It is also contemplated that at the end of the second period of time T2, the intensity of light may be increased to an intensity that is different than the first intensity I1.

The illumination scheme of FIG. 1A provides pulses of light that sharply increase the intensity of light at the beginning of the second period of time T2, which provides a "lights on" NIF response. In contrast, the illumination scheme of FIG. 1B provides "negative" pulses of light that sharply decrease the intensity of light at the beginning of the second period of time T2, which provides a "lights off' NIF response.

FIG. 1C is a graph of an illumination scheme in accordance with an exemplary embodiment of the disclosed concept. In FIG. 1C, the vertical axis represents intensity of illumination and the horizontal axis represents time. Referring to FIG. 1C, light is provided at the first intensity I1 for the first period of time T1. The light provided during the first period of time T1 is a background illumination and may be, without limitation, white light.

At the end of the first period of time T1, the intensity of the light is increased substantially instantaneously to an intensity that is factor F times the first intensity I1. Over the second period of time T2, the intensity of light gradually returns to the first intensity I1. It is also contemplated that over the second period of time T2, the intensity of light may be increased to an intensity that is different than the first intensity I1.

The sharp increase in the intensity of light at the beginning of the second period of time T2 provides a "lights on" NIF response while the gradual decrease in the intensity of light over the second period of time T2 diminishes the "lights off' NIF response.

FIG. 1D is a graph of an illumination scheme in accordance with an exemplary embodiment of the disclosed concept. The illumination scheme of FIG. 1D is similar to the illumination scheme of FIG. 1C, except that in the illumination scheme of FIG. 1D, the intensity of light gradually increases over the second period of time T2 and then sharply decreases at the end of the second period of time T2.

The gradual increase in the intensity of light over the second period of time diminishes a "lights on" NIF response while the sharp decrease in the intensity of light at the end of the second period of time T2 provides a "lights off' NIF response. Thus, the illumination scheme of FIG. 1C may be particularly suitable to suppress melatonin production while the illumination scheme of FIG. 1D may be particularly suitable to stimulate melatonin production.

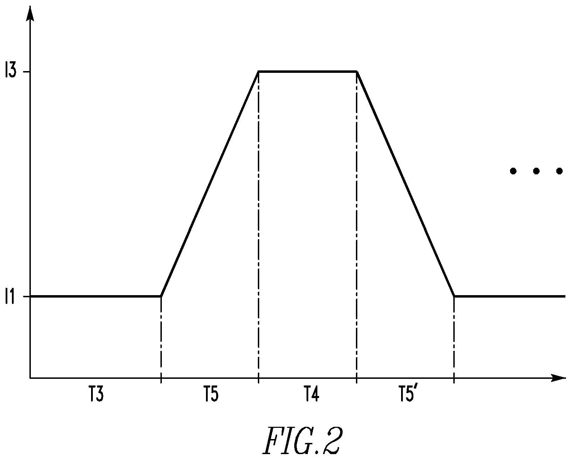

FIG. 2 is a graph of an illumination scheme in accordance with another exemplary embodiment of the disclosed concept. In FIG. 2, the vertical axis represents intensity of illumination and the horizontal axis represents time. Referring to FIG. 2, the illumination scheme begins with providing light at the first intensity I1 for a third period of time T3. Then, over a first transitional period of time T5, the intensity of the light is gradually increased (e.g., without limitation, ramps) to a third intensity I3. The light is maintained at the third intensity 13 for a fourth period of time T4. During a second transitional period of time T5', the intensity of the light is gradually reduced to the first intensity I1. It is contemplated that the intensity of the light may also return to an intensity that is different than the first intensity I1.

The third intensity 13 may be, without limitation, about equal to or greater than 24 times greater than first intensity I1. The first and second transitional periods of time T5 and T5' may each be, without limitation, within a range of about 0.1 seconds to about 150 seconds. In some exemplary embodiments of the disclosed concept, the third period of time T3 may be, without limitation, about 30 seconds. In some exemplary embodiments of the disclosed concept, the fourth period of time T4 may be, without limitation, about 10 seconds.

Experimentation has shown that SCN neurons have responded well to ramps during transitional time period T5. Large responses came from light targeting all photoreceptors. Large responses also came from shorter length of transitional time period T5. Ramping up intensity during the transitional time period T5 induces a strong transient excitation. Additionally, an increased response is associated with the change in steady state illuminance. The gradual change in intensity during transitional time period T5 elicits an enhanced response from ipRGCs.

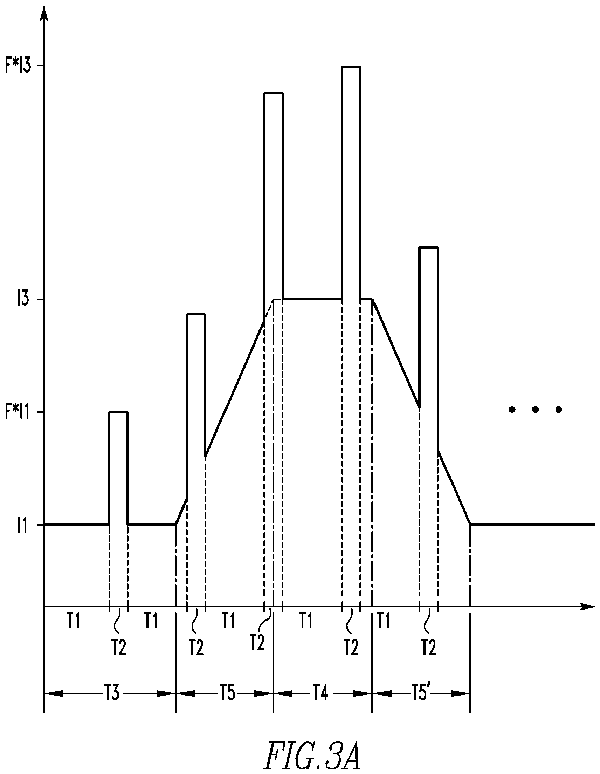

FIG. 3A is a graph of an illumination scheme in accordance with another exemplary embodiment of the disclosed concept. In FIG. 3A, the vertical axis represents intensity and the horizontal axis represents time. The illumination scheme of FIG. 3A combines the illumination schemes of FIGS. 1A and 2 to elicit enhanced responses from both cones and ipRGCs. That is, the illumination scheme of FIG. 1A is overlain on the illumination scheme of FIG. 2.

In more detail, light is provided at the first intensity I1 for the third time period T3. The intensity of the light gradually increases to the third intensity I3 during the first transitional time period T5, and is maintained at the third intensity I3 for the fourth time period T4. The intensity of the light is then gradually decreased to the first intensity 11 during the second transitional time period T5'. Pulses spaced apart by the first time period T1 are also provided. The pulses are each maintained for the second time period T2. The intensity of the pulses are factor F times the intensity of light immediately preceding the pulse.

The illumination scheme of FIG. 3A, by combining both gradual and abrupt changes in intensity of light, elicits enhanced NIF responses from both the cones and ipRGCs.

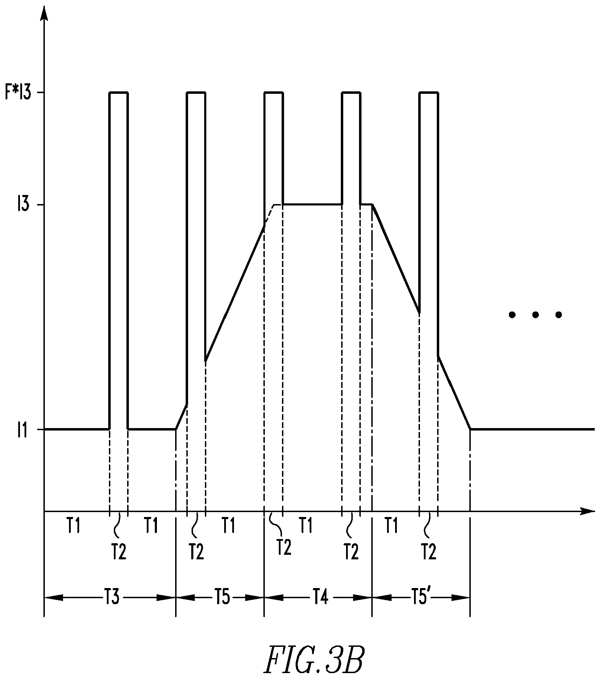

FIG. 3B is a graph of an illumination scheme in accordance with another exemplary embodiment of the disclosed concept. The illumination scheme of FIG. 3B is similar to the illumination scheme of FIG. 3A, except that in the illumination scheme of FIG. 3B, all of the pulses have the same intensity rather than varying intensities. The illumination scheme of FIG. 3B provides similar enhanced NIF responses as the illumination scheme of FIG. 3A, except that the illumination scheme of FIG. 3B may be easier to practically implement.

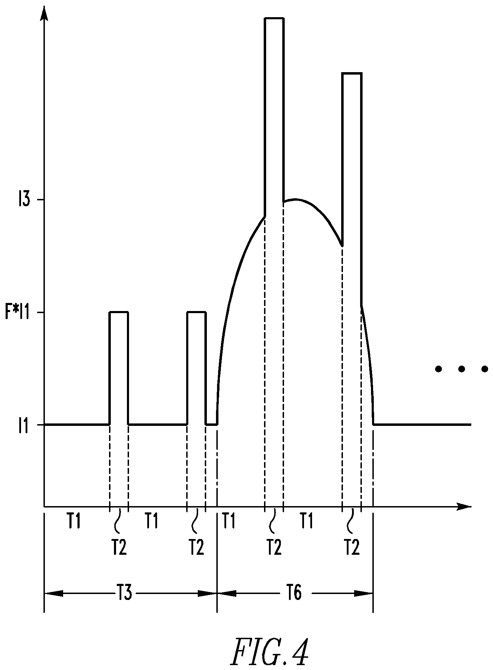

FIG. 4 is a graph of an illumination scheme in accordance with another exemplary embodiment of the disclosed concept. In FIG. 4, the vertical axis represents intensity of illumination and the horizontal axis represents time. The illumination scheme of FIG. 4 generally provides light at the first intensity I1 for the third period of time T3. Then in a sinusoidal manner over a sixth period of time T6, the light gradually increases to the third intensity I3 and then gradually decreases back to the first intensity I1. It is also contemplated that the intensity of light may return to an intensity that is different than the first intensity I1. In some exemplary embodiments of the disclosed concept, the sixth period of time T6 may be, without limitation, within a range of about 0.2 seconds to about 300 seconds. The illumination scheme of FIG. 1A is overlain on this pattern. That is, pulses spaced apart by the first period of time T1 are provided. The pulses have intensities of factor F times the intensity of light immediately preceding the pulse.

The illumination scheme of FIG. 4, like the illumination scheme of FIG. 3A, also elicits enhanced NIF responses from both the cones and the ipRGCs.

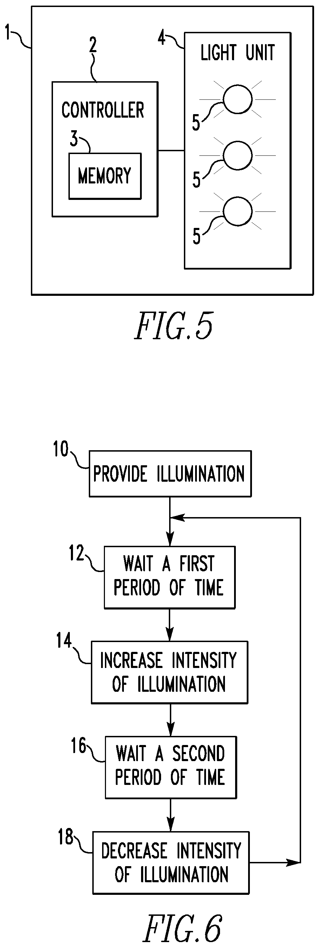

Referring to FIG. 5, an illumination device 1 in accordance with an exemplary embodiment of the disclosed concept is shown. Illumination device includes a controller 2 and a light unit 4. It is contemplated that illumination device 1 may be embodied in, without limitation, personal devices such as alarm clocks or energy lights, or facility light in places such as, without limitation, schools, healthcare facilities, or offices.

Controller 2 may be, for example, a microprocessor, a microcontroller, or some other suitable processing device. Controller 2 includes memory 3 which provides a storage medium for data and software executable by controller 2. It is also contemplated that memory 3 may be operatively connected to controller 2 rather than included in controller 2 without departing from the scope of the disclosed concept. Memory 3 can be any one or more of a variety of types of internal and/or external storage media such as, without limitation, RAM, ROM, EPROM(s), EEPROM(s), FLASH, and the like that provide a storage register, i.e., a machine readable medium, for data storage such as in the fashion of an internal storage area of a computer, and can be volatile memory or nonvolatile memory. Memory 3 may also be a removable device that is able to be removed from controller 2.

Light unit 4 is structured to provide an illumination output and includes a number of light sources 5. Light sources 5 may include, for example, fluorescent, incandescent, halogen, high intensity discharge (HID), light emitting diodes (LEDs) or other light sources.

Controller 2 is structured to control light unit 4 to turn on, turn off, and vary the intensity or other characteristics of light produced by light sources 5. To this end, the controller 2 is structured to include one or more programs to control light unit 4 to produce one or more of the illumination schemes depicted in FIGS. 1-4. Controller 2 may be structured to implement any of the methods that will be described hereinafter with reference to FIGS. 6-9.

FIG. 6 is a flowchart of a method of providing an illumination scheme in accordance with an exemplary embodiment of the disclosed concept. The method of providing an illumination scheme shown in FIG. 6 may be employed to produce the illumination scheme shown in FIG. 1A. It is contemplated that the method of providing an illumination scheme shown in FIG. 6 may be implemented in, for example, controller 2 to control illumination device 1 to produce the illumination scheme shown in FIG. 1A.

At 10, illumination is provided. Illumination may be provided at, for example, the first intensity I1. At 12, the first period of time T1 is allowed to pass. The illumination is continued to be provided while the first period of time T1 passes. At 14, the intensity of illumination is increased. The intensity of illumination is increased to, for example, the factor F times the first intensity I1. At 16, the second period of time T2 is allowed to pass. At 18, the intensity of illumination is decreased to, for example, the first intensity I1. The method returns to 12 and may repeat as often as desired.

It is contemplated that modifications may be made to the method of FIG. 6 in order to produce the illumination schemes of FIGS. 1B, 1C, or 1D, without departing from the scope of the disclosed concept.

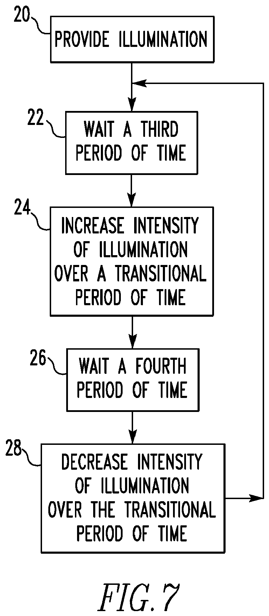

FIG. 7 is a flowchart of a method of providing an illumination scheme in accordance with an exemplary embodiment of the disclosed concept. The method of providing an illumination scheme shown in FIG. 7 may be employed to produce the illumination scheme shown in FIG. 2. It is contemplated that the method of providing an illumination scheme shown in FIG. 7 may be implemented in, for example, controller 2 to control illumination device 1 to produce the illumination scheme shown in FIG. 2.

At 20, illumination is provided. Illumination may be provided at, for example, the first intensity I1. At 22, the third period of time T3 is allowed to pass. The illumination is continued to be provided while the third period of time T3 passes. At 24, the intensity of illumination is gradually increased over the first transitional period of time T5 to, for example, the third intensity I3. At 26, the fourth period of time T4 is allowed to pass. At 28, the intensity of illumination is gradually decreased over the second transitional period of time T5' to, for example, the first intensity I1. The method returns to 22 and may repeat as often as desired.

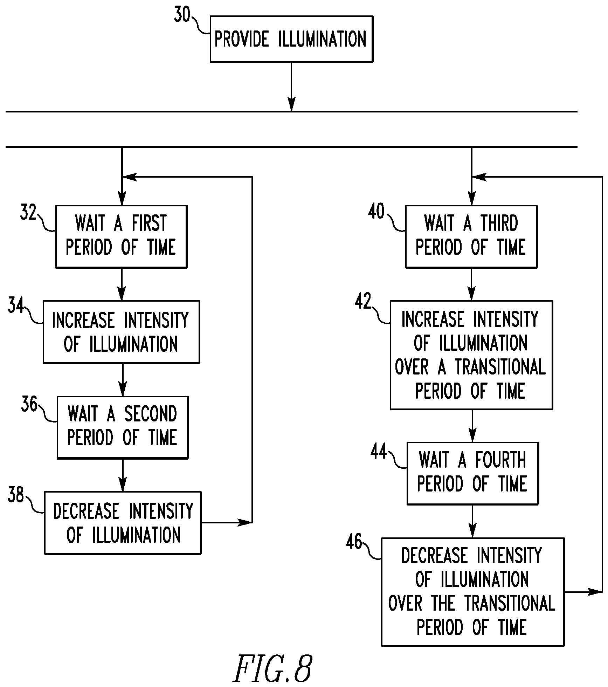

FIG. 8 is a flowchart of a method of providing an illumination scheme in accordance with an exemplary embodiment of the disclosed concept. The method of providing an illumination scheme shown in FIG. 8 may be employed to produce the illumination scheme shown in FIG. 3A or 3B. It is contemplated that the method of providing an illumination scheme shown in FIG. 8 may be implemented in, for example, controller 2 to control illumination device 1 to produce the illumination scheme shown in FIG. 3A or 3B.

At 30, illumination is provided. Illumination may be provided at, for example, the first intensity I1. At 32, the first period of time T1 is allowed to pass. The illumination is continued to be provided while the first period of time T1 passes. At 34, the intensity of illumination is substantially instantaneously increased. The intensity of illumination is increased by, for example, factor F times the intensity immediately preceding the increase (such as in the illumination scheme of FIG. 3A) or factor F times an intensity such as the third intensity 13 (such as in the illumination scheme of FIG. 3B). At 36, the second period of time T2 is allowed to pass. At 38, the intensity of illumination is substantially instantaneously decreased by, for example, about the amount it was increased by at 34. The method returns to 32 and may repeat as often as desired.

At 40, the third period of time T3 is allowed to pass. The illumination is continued to be provided while the third period of time T3 passes. At 42, the intensity of illumination is gradually increased over the transitional period of time T5 to, for example, the third intensity 13. At 44, the fourth period of time T4 is allowed to pass. At 46, the intensity of illumination is gradually decreased over the transitional period of time T5' to, for example, the first intensity I1. It is also contemplated that the intensity of light may be decreased to an intensity that is different than the first intensity I1. The method returns to 40 and may repeat as often as desired.

Operations 32, 34, 36 and 38 are performed in parallel with operations 40, 42, 44 and 46. The result is a series of pulses of increased intensity of illumination that overlay a gradual ramping up and ramping down of intensity of illumination, such as the illumination schemes shown in FIGS. 3A or 3B.

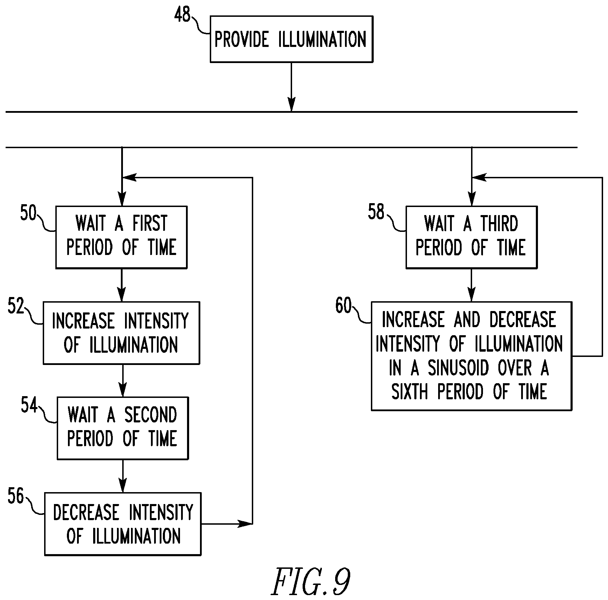

FIG. 9 is a flowchart of a method of providing an illumination scheme in accordance with an exemplary embodiment of the disclosed concept. The method of providing an illumination scheme shown in FIG. 9 may be employed to produce the illumination scheme shown in FIG. 4. It is contemplated that the method of providing an illumination scheme shown in FIG. 9 may be implemented in, for example, controller 2 to control illumination device 1 to produce the illumination scheme shown in FIG. 4.

At 48, illumination is provided. Illumination may be provided at, for example, the first intensity I1. At 50, the first period of time T1 is allowed to pass. The illumination is continued to be provided while the first period of time T1 passes. At 52, the intensity of illumination is substantially instantaneously increased. The intensity of illumination is increased by, for example, factor F times the intensity of light immediately preceding the increase. At 54, the second period of time T2 is allowed to pass. At 56, the intensity of illumination is substantially instantaneously decreased by, for example, about the amount it was increased by at 52. The method returns to 50 and may repeat as often as desired.

At 58, the third period of time T3 is allowed to pass. The illumination is continued to be provided while the third period of time T3 passes. At 60, the intensity of illumination is gradually increased to the third intensity 13 and gradually decreased to, for example, the first intensity I1, in a sinusoidal manner over the sixth period of time T6. The method returns to 58 and may repeat as often as desired.

Operations 50, 52, 54 and 56 are performed in parallel with operations 58 and 60. The result is a series of pulses of increased intensity of illumination that overlay a periodic sinusoidal ramping up and ramping down of intensity of illumination, such as the illumination scheme shown in FIG. 4.

The disclosed concept can also be embodied as computer readable codes on a tangible, non-transitory computer readable recording medium. The computer readable recording medium is any data storage device that can store data which can be thereafter read by a computer system. Non-limiting examples of the computer readable recording medium include read-only memory (ROM), non-volatile random-access memory (RAM), CD-ROMs, magnetic tapes, floppy disks, disk storage devices, and optical data storage devices.

It is contemplated that the disclosed concept may be employed in a variety of applications such as, without limitation, personal devices such as alarm clocks or energy lights, or facility light in places such as, without limitation, schools, healthcare facilities, or offices.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

## Claims

1. An illumination device (1) for generating a non-image forming response in the supra chiasmatic nuclei (SCN) of human beings, the illumination device comprising:
a light unit (4) structured to provide an illumination output; and
a controller (2) structured to control said light unit to provide the illumination output at a first intensity and to wait a first period of time, increase intensity of the illumination output to at least a factor times the first intensity, wait a second period of time, and decrease intensity of the illumination,
wherein the factor is at least 1.25,
and
wherein the controller is structured to control said light unit to increase intensity of the illumination output substantially instantaneously or to control said light unit to decrease intensity of the illumination output substantially instantaneously to thereby elicit a non-image forming response from cone photoreceptors;
**characterised in that**:
the illumination output during the first period of time and the illumination output during the second period of time have the same spectral composition.

2. The illumination device of claim 1, wherein the factor is at least 8.

3. The illumination device of claim 1, wherein the factor is at least 14.

4. The illumination device of claim 1, wherein the second period of time is within a range of about 0.1 seconds to about 60 seconds and wherein the first period of time is at least three times greater than the second period of time.

5. The illumination device of claim 1, wherein the controller is structured to control the light unit to repetitively perform operation (a) and one of operations (b) and (c) in parallel, wherein (a), (b) and (c) are:
(a) wait the first period of time, increase intensity of the illumination output to at least the factor times the first intensity, wait the second period of time, and decrease intensity of the illumination output;
(b) wait a third period of time, increase intensity of the illumination output over a first transitional period of time, wait a fourth period of time, and decrease intensity of the illumination output over a second transitional period of time; and
(c) wait a third period of time and gradually increase and gradually decrease intensity of the illumination output in a substantially sinusoidal form over a sixth period in time.

6. The illumination device of claim 5 wherein the increased illumination output comprises light of a wavelength in a ipRGC photoreceptor sensitivity range.

7. The illumination device of claim 5 wherein the first and second transitional periods of time are each within a range of about 0.1 seconds to about 150 seconds

8. A computer program product comprising computer program code enabling a controller of the illumination device of any of the claims 1-8, when executing said computer program code, to provide an illumination scheme for generating a non-image forming response in the supra chiasmatic nuclei (SCN) of human beings, wherein the computer program code enables the controller to:
control a light unit to provide an illumination output at a first intensity;
wait a first period of time;
increase intensity of the illumination output to at least a factor times the first intensity;
wait a second period of time; and
decrease intensity of the illumination output,
wherein the factor is at least 1.25,
wherein the illumination output during the first period of time and the illumination output during the second period of time have the same spectral composition, and
wherein the intensity of the illumination output is increased substantially instantaneously or decreased substantially instantaneously to thereby elicit a non-image forming response from cone photoreceptors.

9. The computer program product of claim 8, wherein the computer program code enables the controller to control the second period of time to be within a range of about 0.1 seconds to about 60 seconds and the first period of time to be at least three times greater than the second period of time.

10. The computer program product of claim 8, wherein the computer program code enables the controller to control the light unit to repetitively perform operation (a) and one of operations (b) and (c) in parallel, wherein (a), (b) and (c) are:
(a) wait the first period of time, increase intensity of the illumination output to at least the factor times the first intensity, wait the second period of time, and decrease intensity of the illumination output;
(b) wait a third period of time, increase intensity of the illumination output over a first transitional period of time, wait a fourth period of time, and decrease intensity of the illumination output over a second transitional period of time; and
(c) wait a third period of time and gradually increase and gradually decrease intensity of the illumination output in a substantially sinusoidal form over a sixth period in time, and
wherein the increased illumination output comprises light of a wavelength in a ipRGC photoreceptor sensitivity range.

11. A non-transitory computer readable medium stored thereon the computer program product of any of the claims 8-10.

## Patentansprüche

1. Beleuchtungsvorrichtung (1) zur Generierung einer nichtbilderzeugenden Reaktion in dem Nucleus suprachiasmaticus (SCN) von Menschen, wobei die Beleuchtungsvorrichtung umfasst:
eine Lichteinheit (4), die so eingerichtet ist, dass sie eine Beleuchtungsausgabe bereitstellt; sowie
eine Steuereinheit (2), die so eingerichtet ist, dass sie diese Lichteinheit so steuert, dass diese die Beleuchtungsausgabe bei einer ersten Intensität bereitstellt und einen ersten Zeitraum abwartet, die Intensität der Beleuchtungsausgabe auf zumindest einen Faktor mal der ersten Intensität erhöht, einen zweiten Zeitraum abwartet und die Intensität der Beleuchtung verringert,
wobei der Faktor mindestens 1,25 beträgt, und
wobei die Steuereinheit so eingerichtet ist, dass sie diese Lichteinheit so steuert, dass diese die Intensität der Beleuchtungsausgabe im Wesentlichen unverzüglich erhöht oder diese Lichteinheit so steuert, dass diese die Intensität der Beleuchtungsausgabe im Wesentlichen unverzüglich verringert, um dadurch eine nichtbilderzeugende Reaktion von Zapfen-Photorezeptoren hervorzurufen;
**dadurch gekennzeichnet, dass**:
die Beleuchtungsausgabe während des ersten Zeitraums und die Beleuchtungsausgabe während des zweiten Zeitraums die gleiche spektrale Zusammensetzung aufweisen.

2. Beleuchtungsvorrichtung nach Anspruch 1, wobei der Faktor mindestens 8 beträgt.

3. Beleuchtungsvorrichtung nach Anspruch 1, wobei der Faktor mindestens 14 beträgt.

4. Beleuchtungsvorrichtung nach Anspruch 1, wobei der zweite Zeitraum innerhalb eines Bereichs von etwa 0,1 Sekunden bis etwa 60 Sekunden liegt, und wobei der erste Zeitraum mindestens dreimal größer als der zweite Zeitraum ist.

5. Beleuchtungsvorrichtung nach Anspruch 1, wobei die Steuereinheit so eingerichtet ist, dass sie die Lichteinheit so steuert, dass diese Vorgang (a) wiederholend und Vorgang (b) oder (c) parallel ausführt, wobei (a), (b) und (c) beinhalten, dass sie:
(a) den ersten Zeitraum abwartet, die Intensität der Beleuchtungsausgabe auf zumindest den Faktor mal der ersten Intensität erhöht, den zweiten Zeitraum abwartet und die Intensität der Beleuchtungsausgabe verringert;
(b) einen dritten Zeitraum abwartet, die Intensität der Beleuchtungsausgabe über einen ersten Übergangszeitraum erhöht, einen vierten Zeitraum abwartet und die Intensität der Beleuchtungsausgabe über einen zweiten Übergangszeitraum verringert; und
(c) einen dritten Zeitraum abwartet und die Intensität der Beleuchtungsausgabe in einer im Wesentlichen sinusförmigen Form über einen sechsten Zeitraum graduell erhöht und graduell verringert.

6. Beleuchtungsvorrichtung nach Anspruch 5, wobei die erhöhte Beleuchtungsausgabe Licht einer Wellenlänge in einem ipRGC-Photorezeptoren-Empfindlichkeitsbereich umfasst.

7. Beleuchtungsvorrichtung nach Anspruch 5, wobei der erste und zweite Übergangszeitraum jeweils innerhalb eines Bereichs von etwa 0,1 Sekunden bis etwa 150 Sekunden liegen.

8. Computerprogrammprodukt mit Computerprogrammcode, um eine Steuereinheit der Beleuchtungsvorrichtung nach einem der Ansprüche 1-8 in die Lage zu versetzen, bei Ausführung dieses Computerprogrammcodes ein Beleuchtungsschema zur Generierung einer nichtbilderzeugenden Reaktion in dem Nucleus suprachiasmaticus (SCN) von Menschen bereitzustellen, wobei der Computerprogrammcode es der Steuereinheit ermöglicht:
eine Lichteinheit so zu steuern, dass diese die Beleuchtungsausgabe bei einer ersten Intensität bereitstellt;
einen ersten Zeitraum abwartet,
die Intensität der Beleuchtungsausgabe auf zumindest einen Faktor mal der ersten Intensität erhöht;
einen zweiten Zeitraum abwartet; und
die Intensität der Beleuchtungsausgabe verringert,
wobei der Faktor mindestens 1,25 beträgt,
wobei die Beleuchtungsausgabe während des ersten Zeitraums und die Beleuchtungsausgabe während des zweiten Zeitraums die gleiche spektrale Zusammensetzung aufweisen, und
wobei die Intensität der Beleuchtungsausgabe im Wesentlichen unverzüglich erhöht oder im Wesentlichen unverzüglich verringert wird, um dadurch eine nichtbilderzeugende Reaktion von Zapfen-Photorezeptoren hervorzurufen.

9. Computerprogrammprodukt nach Anspruch 8, wobei der Computerprogrammcode die Steuereinheit in die Lage versetzt, den zweiten Zeitraum so zu steuern, dass dieser innerhalb eines Bereichs von etwa 0,1 Sekunden bis etwa 60 Sekunden liegt und der erste Zeitraum mindestens dreimal größer als der zweite Zeitraum ist.

10. Computerprogrammprodukt nach Anspruch 8, wobei der Computerprogrammcode die Steuereinheit in die Lage versetzt, die Lichteinheit so zu steuern, dass diese Vorgang (a) wiederholend und Vorgang (b) oder (c) parallel ausführt, wobei (a), (b) und (c) beinhalten, dass sie:
(a) den ersten Zeitraum abwartet, die Intensität der Beleuchtungsausgabe auf zumindest den Faktor mal der ersten Intensität erhöht, den zweiten Zeitraum abwartet und die Intensität der Beleuchtungsausgabe verringert;
(b) einen dritten Zeitraum abwartet, die Intensität der Beleuchtungsausgabe über einen ersten Übergangszeitraum erhöht, einen vierten Zeitraum abwartet und die Intensität der Beleuchtungsausgabe über einen zweiten Übergangszeitraum verringert; sowie
(c) einen dritten Zeitraum abwartet und die Intensität der Beleuchtungsausgabe in einer im Wesentlichen sinusförmigen Form über einen sechsten Zeitraum graduell erhöht und graduell verringert, und
wobei die erhöhte Beleuchtungsausgabe Licht einer Wellenlänge in einem ipRGC-Photorezeptoren-Empfindlichkeitsbereich umfasst.

11. Nichttransitorisches, computerlesbares Medium mit dem darauf gespeicherten Computerprogrammprodukt nach einem der Ansprüche 8-10.

## Revendications

1. Dispositif d'éclairage (1) pour générer une réponse formant une non-image dans les noyaux suprachiasmatiques (NSC) d'êtres humains, le dispositif d'éclairage comprenant :
une unité de lumière (4) structurée pour fournir une sortie d'éclairage ; et
un dispositif de commande (2) structuré pour commander ladite unité de lumière pour fournir la sortie d'éclairage à une première intensité et pour attendre une première période de temps, augmenter l'intensité de la sortie d'éclairage à au moins un facteur fois la première intensité, attendre une deuxième période de temps et diminuer l'intensité de l'éclairage,
dans lequel le facteur est d'au moins 1,25,
dans lequel le dispositif de commande est structuré pour commander ladite unité de lumière pour augmenter l'intensité de la sortie d'éclairage de façon sensiblement instantanée ou pour commander ladite unité de lumière pour diminuer l'intensité de la sortie d'éclairage de façon sensiblement instantanée pour ainsi déclencher une réponse formant une non-image à partir de photorécepteurs cônes ;
**caractérisé en ce que** :
la sortie d'éclairage pendant la première période de temps et la sortie d'éclairage pendant la deuxième période de temps ont la même composition spectrale.

2. Dispositif d'éclairage selon la revendication 1, dans lequel le facteur est d'au moins 8.

3. Dispositif d'éclairage selon la revendication 1, dans lequel le facteur est d'au moins 14.

4. Dispositif d'éclairage selon la revendication 1, dans lequel la deuxième période de temps est dans une plage entre environ 0,1 seconde et environ 60 secondes et dans lequel la première période de temps est au moins trois fois plus longue que la deuxième période de temps.

5. Dispositif d'éclairage selon la revendication 1, dans lequel le dispositif de commande est structuré pour commander l'unité de lumière pour exécuter de façon répétée l'opération (a) et l'une des opérations (b) et (c) en parallèle, dans lequel (a), (b) et (c) sont :
(a) attendre la première période de temps, augmenter l'intensité de la sortie d'éclairage à au moins le facteur fois la première intensité, attendre la deuxième période de temps, et diminuer l'intensité de la sortie d'éclairage ;
(b) attendre une troisième période de temps, augmenter l'intensité de la sortie d'éclairage sur une première période de temps de transition, attendre une quatrième période de temps et diminuer l'intensité de la sortie d'éclairage sur une deuxième période de temps de transition ; et
(c) attendre une troisième période de temps et augmenter progressivement et diminuer progressivement l'intensité de la sortie d'éclairage dans une forme sensiblement sinusoïdale sur une sixième période de temps.

6. Dispositif d'éclairage selon la revendication 5 dans lequel la sortie d'éclairage augmentée comprend une lumière d'une longueur d'onde dans une plage de sensibilité de photorécepteur ipRGC.

7. Dispositif d'éclairage selon la revendication 5 dans lequel les première et deuxième périodes de temps de transition sont chacune dans une plage comprise entre environ 0,1 seconde et environ 150 secondes.

8. Produit de programme informatique comprenant un code de programme informatique permettant à un dispositif de commande du dispositif d'éclairage selon l'une quelconque des revendications 1 à 8, lors de l'exécution dudit code de programme informatique, de fournir un schéma d'éclairage pour générer une réponse formant une non-image dans les noyaux suprachiasmatiques (NSC) d'êtres humains, dans lequel le code de programme informatique permet au dispositif de commande de :
commander une unité de lumière pour fournir une sortie d'éclairage à une première intensité ;
attendre une première période de temps ;
augmenter l'intensité de la sortie d'éclairage à au mois un facteur fois la première intensité ;
attendre une deuxième période de temps ; et
diminuer l'intensité de la sortie d'éclairage,
dans lequel le facteur est d'au moins 1,25,
dans lequel la sortie d'éclairage pendant la première période de temps et la sortie d'éclairage pendant la deuxième période de temps ont la même composition spectrale, et
dans lequel l'intensité de la sortie d'éclairage est augmentée de façon sensiblement instantanée ou diminuée de façon sensiblement instantanée pour ainsi déclencher une réponse formant une non-image à partir de photorécepteurs cônes.

9. Produit de programme informatique selon la revendication 8, dans lequel le code de programme informatique permet au dispositif de commande de commander la deuxième période de temps pour être dans une plage entre environ 0,1 seconde et environ 60 secondes et la première période de temps pour être au moins trois fois plus longue que la deuxième période de temps.

10. Produit de programme informatique selon la revendication 8, dans lequel le code de programme informatique permet au dispositif de commande de commander l'unité de lumière pour exécuter de façon répétée l'opération (a) et l'une des opérations (b) et (c) en parallèle, dans lequel (a), (b) et (c) sont :
(a) attendre la première période de temps, augmenter l'intensité de la sortie d'éclairage à au moins le facteur fois la première intensité, attendre la deuxième période de temps, et diminuer l'intensité de la sortie d'éclairage
(b) attendre une troisième période de temps, augmenter l'intensité de la sortie d'éclairage sur une première période de temps de transition, attendre une quatrième période de temps et diminuer l'intensité de la sortie d'éclairage sur une deuxième période de temps de transition ; et
(c) attendre une troisième période de temps et augmenter progressivement et diminuer progressivement l'intensité de la sortie d'éclairage dans une forme sensiblement sinusoïdale sur une sixième période de temps, et
dans lequel la sortie d'éclairage augmentée comprend une lumière d'une longueur d'onde dans une plage de sensibilité de photorécepteur ipRGC.

11. Support non transitoire lisible par ordinateur stockant sur celui-ci le produit de programme informatique selon l'une quelconque des revendications 8 à 10.
